# EUROPÄISCHE PATENTSCHRIFT

(11) **EP 2 049 180 B1**
(45) Veröffentlichungstag und Bekanntmachung des Hinweises auf die Patenterteilung: **12.09.2012**
(21) Anmeldenummer: 07764425.0
(22) Anmeldetag: 04.07.2007
(51) Int. Cl.: A61M 16/01, A61M 16/12

(54) **ATEMGERÄT UND EIN VERFAHREN ZUM BETRIEB HIERZU**
RESPIRATORY DEVICE, AND METHOD FOR THE OPERATION THEREOF
APPAREIL RESPIRATOIRE ET SON PROCÉDÉ DE FONCTIONNEMENT

(30) Priorität: 05.07.2006 DE 102006032498
(43) Veröffentlichungstag der Anmeldung: 22.04.2009
(73) Patentinhaber: F. Stephan Gmbh Medizintechnik, 56412 Gackenbach (DE)
(72) Erfinder: MAINUSCH, Georg, 56076 Koblenz (DE)
(74) Vertreter: Grüneberg, Marcus
(86) Internationale Anmeldenummer: PCT/DE2007/001177
(87) Internationale Veröffentlichungsnummer: WO 2008/003300

(56) Entgegenhaltungen:
- EP-A- 0 916 357
- WO-A-01/07108
- DE-A1-102004 011 907
- GB-A- 2 335 604
- US-A- 4 791 922
- US-A- 5 520 172
- US-A- 5 810 002

## Beschreibung

Die Erfindung betrifft ein Atemgerät, insbesondere ein kombiniertes Narkose- und Therapiebeatmungsgerät für Patienten zur therapeutischen und operativen Anwendung sowie ein Verfahren zum Betrieb hierzu.

Narkose- und Therapiebeatmungsgeräte werden als offene, halboffene, halbgeschlossene und quasigeschlossene Atemgeräte betrieben. Den aus dem Stand der Technik vorbekannten Atemgeräten ist immanent, dass diese in Abhängigkeit ihrer Konstruktion überschüssige und kostenintensive Anästhesiegase verlieren.

Aus dem Stand der Technik ist dazu die DE 10 2004 011 907 A1 vorbekannt, in welcher ein gattungsgemäßes Narkosebeatmungsgerät offenbart ist. Das Narkosebeatmungsgerät weist u. a. einen Balg, der sich innerhalb eines Domes befindet, Einwegventile, einen CO₂-Absorber, ein Umschaltventil, ein Y-Stück sowie eine Frischgasbereitstellung auf. Kennzeichnend für diese Erfindung ist, dass das Antriebsgas für den Dom oder für die Steuerkammer eines Überströmventils von einem Doppelventil, umfassend ein Inspirationsventil und ein Exspirationsventil, bereitgestellt wird. Das Doppelventil wird im inspiratorischen Zweig von einem positiven Vordruck und im exspiratorischen Zweig von einem negativen Vordruck gespeist. Der besondere Nachteil dieser Erfindung ist, dass überschüssiges Anästhesiegas ungenutzt in die zentrale Narkoseabsaugung ausgestoßen wird.

Die Aufgabe der Erfindung besteht nunmehr darin, ein Atemgerät, insbesondere ein kombiniertes Narkose- und Therapiebeatmungsgerät vorzuschlagen, mit dem eine effizientere Nutzung des dem Patienten über das Atemgerät zuzuführenden Anästhesiegases erzielt werden kann.

Erfindungsgemäß weist das Atemgerät, insbesondere ein kombiniertes Narkose- und Therapiebeatmungsgerät, zumindest eine Frischgasbereitstellung, eine Antriebsgasbereitstellung, ein Y-Stück mit einem Inspirationszweig und einem Exspirationszweig sowie zugehörige Steuer- und Regeleinrichtungen auf, wobei zum Zwecke der Rückgewinnung und Rückführung von überschüssigen Anästhesiegas ein im Exspirationszweig platziertes steuerbares Umschaltventil mit einem Rückführungsströmungspfad und einem Fortgasströmungspfad sowie ein im Rückführungsströmungspfad platziertes und in den Inspirationszweig mündendes Gasreservoir vorgesehen sind, wobei dem Gasreservoir in Gasströmungsrichtung ein Frischgasentkopplungsventil nachgeschaltet ist, welches sowohl mit dem Gasreservoir als auch mit der Frischgasbereitstellung gekoppelt ist.

Bei einer vorteilhaften Weiterbildung der Erfindung ist zwischen dem Gasreservoir und dem Frischgasentkopplungsventil ein Mischpunkt platziert, welcher sowohl mit dem Gasreservoir als auch mit der Frischgasbereitstellung verbunden ist.

Das vorzugsweise unmittelbar an der äußeren Grenze des Exspirationszweiges angeordnete Umschaltventil ist zum Zwecke der Umschaltung des Atemgerätes in ein halbgeschlossenes oder ein geschlossenes System zwischen seinen Endlagen positionierbar und steuerbar ausgebildet. Das Umschaltventil kann unter Verwendung der Regel- und Steuereinrichtungen derart angesteuert werden, dass entweder der Fortgasströmungspfad oder der Rückführungsströmungspfad freigegeben wird. In der Praxis wird das Frischgasentkopplungsventil in Abhängigkeit der Atemphasen des Patienten gesteuert wird, wobei in der Inspirationsphase das Frischgasentkopplungsventil geschlossen ist, so dass das von der Frischgasbereitstellung gelieferte Frischgas ausschließlich in das Gasreservoir strömen kann. Der Strömungsweg in das weitere Atemgerät ist dabei unterbunden, da es sonst zu einem konkurrierenden Verhalten von Gasreservoir und Patientenlunge führen würde. In der Exspirationsphase hingegen ist das Frischgasentkopplungsventil geöffnet, so dass das von der Frischgasbereitstellung gelieferte Frischgas, das im Gasreservoir bevorrate Gas und das rückströmende und wiedergewonnene Anästhesiegas in das Atemgerät strömen kann.

Bei einigen Betriebszuständen des Atemgerätes kann es vorkommen, dass sich ein Überschuss von Anästhesiegas im Atemgerät befindet und dabei die vom Arzt eingestellten Druckparameter theoretisch unzulässigerweise überschritten werden. In diesem Fall wäre der von der Frischgasbereitstellung gelieferte Frischgasstrom zu hoch und das Atemgerät würde über das Umschaltventil überschüssiges Anästhesiegas in den Fortgasströmungspfad ausstoßen. Das als regulierender Speicher ausgebildete erfindungsgemäße Gasreservoir dient deshalb zum Bevorraten von nicht optimal ausgeregelten Frischgasströmen hinsichtlich Volumenstrom, Druck und Konzentration sowie von dem über den Rückführungsströmungspfad wiedergewonnenen Anästhesiegas. Darüber hinaus ist das Gasreservoir zum Zwecke der Beatmung durch Handkraft als Handbeatmungsbeutel ausgebildet.

Erfindungsgemäß ist zur Absorbierung des CO₂ des in das Atemgerät einströmenden Frischgases und/oder des rückströmenden und wiedergewonnenen Anästhesiegases ein CO₂-Absorber vorgesehen. Somit ist sichergestellt, dass vor der Applizierung des Anästhesiegases am Patienten im Inspirationszweig keine Spurenelemente von CO₂ zu verzeichnen sind. Erfindungsgemäß werden das in das Atemgerät einströmende Frischgas und das rückströmende und wiedergewonnene Anästhesiegas sowohl am Mischpunkt ihrer Strömungswege als auch im Gasreservoir einer Durchmischung unterzogen, bevor das durchgemischte Gas über den CO₂-Absorber dem Inspirationszweig zugeführt wird. Die erneute Nutzung des wiedergewonnen und rückgeführten Anästhesiegases führt besonders vorteilhaft zu einer Reduzierung der Anästhesiemedikamente.

Bei stetigen Strömen der Frischgaszuführung kommt es üblicherweise auch zu einer Volumenzunahme im Atemgerät und damit auch zu einer Volumenzunahme im Gasreservoir. Mit dem Erreichen eines maximalen Erschöpfungsgrad des Gasreservoirs ist ein geringer Druckanstieg im Atemgerät zu verzeichnen, was sich jedoch sehr ungünstig auf den durch das Atemgerät mit Anästhesiegas versorgten Patienten auswirkt.

Mit dem erfindungsgemäßen Verfahren zum Betrieb des Atemgerätes, insbesondere ein kombiniertes Narkose- und Therapiebeatmungsgerät, kann dieser unerwünschte und zum Teil auch lebensgefährliche Druckanstieg im Atemgerät vermieden werden. Kennzeichnend für das erfindungsgemäße Verfahren zum Betrieb des Atemgerätes ist, dass der als Indikator für den Füllungsgrad im Atemgerät verwendete exspiratorische Druck (PEEP) des Atemgerätes geregelt wird, wobei als Stellgrößen der Frischgaszustrom - definiert als Stellung eines Gasmischers - sowie der Fortgasstrom - definiert als Stellung eines Umschaltventils und eines PEEP-Ventils - eingesetzt werden.

Die Regel- und Steuereinrichtungen des Atemgerätes überwachen hierzu permanent den PEEP der Lunge des Patienten Atemzug für Atemzug. Der PEEP ist proportional zum Druck im Gasreservoir und damit auch zum Erschöpfungsgrad des Gasreservoirs. Steigt der anfänglich stetige PEEP plötzlich an, so wird durch die Regel- und Steuereinrichtung des Atemgerätes ein Steuersignal zur stufenweisen Frischgasreduzierung generiert, da der hohe Frischgaszustrom zu einem Ansteigen des PEEP geführt hat. Kann der hohe PEEP nicht in einer definierten Zeit auf einen Sollwert abgebaut werden, so wird das Umschaltventil zur Freigabe des Fortgasströmungspfads geöffnet. Für ungefähr ein oder zwei maschinelle Atemzüge wird nun das Umschaltventil gegen die Atmosphäre geöffnet. Der damit verbundene Druckausgleich am Gasreservoir führt zu einer Zunahme der Volumenkapazität des Atemgerätes. Somit wird der erfassbare Erschöpfungsgrad des Gasreservoirs zur Steuerung des an den Rückstrom des wiedergewonnenen Anästheslegases angepassten Frischgaszustroms eingesetzt.

Bei einer vorteilhaften Ausgestaltung der Erfindung werden zur Regelung der Gaskonzentrationen von Xe und O₂ im Frischgaszustrom die inspiratorische Sauerstoffkonzentration (FiO₂), die inspiratorische Kohlendioxidkonzentration (FiCO₂), die exspiratorische Sauerstoffkonzentration (FetO₂) und die exspiratorische Kohlendioxidkonzentration (FetCO₂) des inspiratorischen bzw. exspiratorischen Atemgases als Regelgrößen für den Gasmischer verwendet. Der Vorteil dieser Regelung besteht darin, dass die Messung des Gases Xe als solche unnötig wird. Eine Zugabe von Xe muss nur noch unstetig, also nach dem Prinzip 0 oder 1 erfolgen. Der elektronische Gasmischer ist erfindungsgemäß derart ausgebildet, dass er das Gas O₂ definiert nach Toleranz und Genauigkeitsangaben in das Atemgerät applizieren kann. Der maschinelle Frischgasstrom kann somit an die genannten Regelgrößen automatisch angepasst werden. Bei einer vorteilhaften Ausgestaltung der Erfindung kann diese Regelung dann selbsttätig in einem vom Arzt zu wählenden Betriebmodus, hier Respiratormodus, erfolgen.

Ein weiterer Vorteil des erfindungsgemäßen Atemgerätes in der Ausbildung als halboffenes Atemgerät besteht darin, dass eine schnelle Spülung des Atemgerätes durch die Freigabe des Fortgasströmungspfades des Umschaltventils, die Steuerung weiterer Umschaltventile - damit Umschaltung in ein halbgeschlossenes Atemgerät - sowie durch die Einstellung einer veränderten Konzentrationsvorgabe von O₂ und Xe am Gasmischer realisiert werden kann. Mit dem Erreichen einer gewünschten Konzentration wird das Umschaltventil wieder zur Freigabe des Rückführungsströmungspfads umgeschaltet, wodurch das Atemgerät wieder geschlossen ist.

Das vorgestellte Atemgerät ist erfindungsgemäß sowohl als Narkose- als auch als Therapiebeatmungsgerät verwendbar, wobei jedoch eine Verwendung als Therapiebeatmungsgerät den Einsatz sehr wertvoller Gase erfordert und die dem Atemgerät zugeführten Atemgase stets mittels des CO₂-Absorbers gereinigt werden müssen.

Die signifikanten Vorteile und Merkmale der Erfindung gegenüber dem Stand der Technik sind im Wesentlichen:
■ Detektierung, Rückgewinnung und Rückführung von überschüssigen Anästhesiegas unter Verwendung eines komplexen Ventilsystems sowie des Gasreservoirs und damit Reduzierung der Anästhesiemedikamente,
■ Regelung des als Indikator für den Füllungsgrad im Atemgerät verwendeten exspiratorischen Drucks (PEEP) des Atemgerätes,
■ Automatische Anpassung des Frischgasstrom in Abhängigkeit der Regelgrößen FiO₂, FiCO₂, FetO₂ und FetCO₂ unter Verzicht auf stetige Xe-Dosierung und
■ Möglichkeit einer schnellen Spülung des Atemgerätes unter Verwendung mehrerer Umschaltventile.

Die Ziele und Vorteile dieser Erfindung sind nach sorgfältigem Studium der nachfolgenden ausführlichen Beschreibung der hier bevorzugten, nicht einschränkenden Beispielausgestaltungen der Erfindung mit den zugehörigen Zeichnungen besser zu verstehen und zu bewerten, von denen zeigen:
- Fig. 1:: halboffenes, halbgeschlossenes und geschlossenes Atemgerät,
- Fig.2:: halbgeschlossenes Atemgerät, Exspirationsphase Respirator-Beatmung,
- Fig. 3:: geschlossenes Atemgerät, Exspirationsphase Respirator-Beatmung,
- Fig. 4:: In - Exspirationsphase Hand-Beatmung und
- Fig. 5:: halboffenes Atemgerät, Exspirationsphase Respirator-Beatmung.

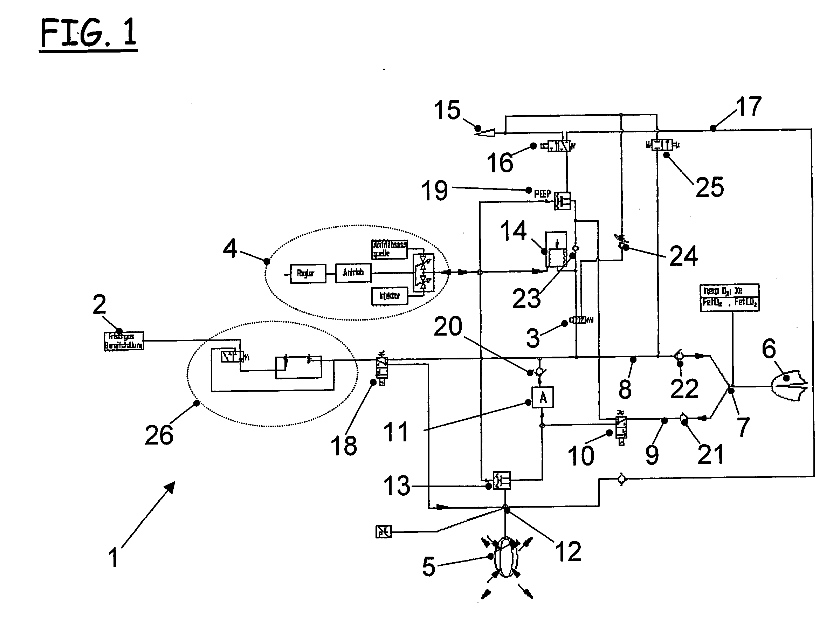

Die Fig. 1 illustriert ein Atemgerät 1, weiches sowohl als halboffenes, halbgeschlossenes und als geschlossenes Atemgerät 1 betrieben werden kann. Das erfindungsgemäße Atemgerät 1 umfasst eine aus dem Stand der Technik vorbekannte Frischgasbereitstellung 2 mit Regler, eine der Frischgasbereitstellung nachgeordnete Druckhalteeinrichtung 26, eine Antriebsgasbereitstellung 4 mit Injektor, Antrieb, Regler und Antriebsgasquelle sowie einen mit einem innerhalb eines Doms 14 angeordneten Balg, ein Y-Stück 7 mit einem Inspirationszweig 8 und einem Exspirationszweig 9 sowie nicht näher dargestellte zugehörige Steuer- und Regeleinrichtungen. Zum Zwecke der Rückgewinnung und Rückführung von überschüssigen Anästhesiegas ist ein komplexes Ventilsystem, umfassend drei Umschaltventil 10, 16 und 18, ein Frischgasentkopplungsventil 13 und eine spezielle Regelung vorgesehen. Mit dem in Strömungsrichtung des Frischgases nach der Frischgasbereitstellung 2 angeordneten Umschaltventil 18 kann das Frischgas zum einen unmittelbar über den Inspirationszweig 8 und über das sich daran anschließende Y-Stück 7 zur Lunge 6 des Patienten zugeführt werden. Die Umschaltung von einem halboffenen Atemgerät in ein halbgeschlossenes Atemgerät erfolgt u. a. auch mit dem Umschaltventil 18. Zum anderen führt ein zweiter Pfad des Umschaltventils 18 zu einem im Rückströmungspfad platzierten Mischpunkt 12. In Strömungsrichtung des Gases ist dem Mischpunkt 12 ein Frischgasentkopplungsventil 13 nachgeschaltet, welches ausgangsseitig mit dem Inspirationszweig verbunden ist. Über diesen zweiten Pfad wird dem Mischpunkt 12 Frischgas und über den Rückführungsströmungspfad 17 wiedergewonnenes Anästhesiegas bedarfsweise zugeführt. Erfindungsgemäß ist des Weiteren ein an der Grenze des Atemgerätes 1 zur Umgebung im Exspirationszweig 9 platziertes steuerbares Umschaltventil 16 vorgesehen. Das Umschaltventil 16 weist den Rückführungsstromungspfad 17 und einen Fortgasströmungspfad 15 auf. Durch die alternative Freigabe des Rückführungsströmungspfades 17 oder des Fortgasstromungspfades 15 kann überschüssiges Anästhesiegas entweder der nicht dargestellten Narkosegasabsaugung zugeleitet oder dem Atemgerät 1 über den Rückführungsströmungspfad 17 und damit dem Mischpunkt 12 zugeleitet werden. Im Bereich des Mischpunktes 12 ist in dem Rückführungsströmungspfad 17 ein Gasreservoir 5 zum Bevorraten von Gas und zum Druckausgleich angeordnet. Bei einer vorteilhaften Ausgestaltung der Erfindung ist das Gasreservoir 5 zum Zwecke der Beatmung durch Handkraft als Handbeatmungsbeutel ausgebildet ist. Das rückgewonnene und rückgeführte Anästhesiegas und das im Gasreservoir 5 bevorrate Anästhesiegas vermischt sich mit dem über das Frischgasentkopplungsventil 13 einströmende Frischgas sowohl am Mischpunkt 12 als auch im Gasreservoir 5. Im Bereich des Gasreservoirs, beispielsweise am Mischpunkt 12, ist ein nicht näher bezeichneter Drucksensor angeordnet, der zur frühzeitigen Erkennung eines Frischgasüberschusses und damit zur optimalen Frischgasanpassung an das Atemsystem eingesetzt wird. Darüber hinaus erfüllt der Drucksensor eine Wamfunktion, sodass bei einem kritischen Zustand auf eine Handbeatmung unter Verwendung des als Handbeatmungsbeutel ausgebildeten Gasreservoirs 5 umgestellt werden kann. Das Frischgasentkopplungsventil 13, das Umschaltventil für die Frischgaszufuhr 18 und das Umschaltventil 16 korrespondieren unter Verwendung der Steuer- und Regeleinrichtung derart, dass in Abhängigkeit des Volumens und der Konzentration des wiedergewonnenen und in das Atemgerät 1 rückgeführten Anästhesiegase das komplementäre Volumen und die zugehörige Konzentration des Frischgases geregelt werden. Der in Strömungsrichtung des "Mischgases" nach dem Mischpunkt 12 angeordnete und mit dem Inspirationszweig 8 gekoppelte CO₂-Absorber 11 absorbiert das im "Mischgas" enthaltene CO₂, so dass dem Patienten nur medizinisch reines Anästhesiegas appliziert wird. Im Exspirationszweig 9 dem Y-Stück 7 unmittelbar nachgeschaltet ist ein weiteres Umschaltventil 10 angeordnet, mit welchem exspiratorischen Narkosegase entweder unmittelbar dem Umschaltventil 16 oder - unter der Voraussetzung der Vermischung mit Frischgas und der Absorbierung von CO₂ mittels des Absorbers 11 - dem Inspirationszweig 8 zugeführt wird. Eine Kemidee der Erfindung ist, dass der als Indikator für den Füllungsgrad im Atemgerät 1 verwendete exspiratorische Druck (PEEP) des Atemgerätes 1 geregelt wird, wobei als Stellgrößen der Frischgaszustrom - definiert als Stellung eines Gasmischers - sowie der Fortgasstrom - definiert als Stellung eines Umschaltventils und eines PEEP-Ventils - eingesetzt werden. Für diesen Zweck ist eine Steuerleitung vorgesehen, die sich zwischen dem Frischgasentkopplungsventil 13 und einem dem Umschaltventil 16 vorgeschalteten Membranventil für die PEEP-Regelung 19 erstreckt. Das Membranventil für die PEEP-Regelung 19 seinerseits weist eine durch den Steuerdruck geregelte Membrane auf, die bei einem zu hohen Überschuss von Anästhesiegases öffnet, so dass das überschüssige Anästhesiegas zum Umschaltventil 16 geleitet wird. Als Regelgrößen für den Gasmischer werden die inspiratorische Sauerstoffkonzentration (FiO₂), die inspiratorische Kohlendioxidkonzentration (FiCO₂), die exspiratorische Sauerstoffkonzentration (FetO₂) und die exspiratorische Kohlendioxidkonzentration (FetCO₂) der inspiratorischen bzw. exspiratorischen Atemgase verwendet, um die erforderliche Gaskonzentrationen von Xe und O₂ im Frischgaszustrom zu ermitteln. Zwischen dem Inspirationszweig 8 und dem Fortgasströmungspfad 15 ist ein weiterer Zweig angeordnet, in welchem ein Notentlüftungsventil 25 mit einem Ansprechdruck von 70 mbar - welches bei zu hohem Druck manuell geöffnet werden kann - platziert ist. Eine schnelle Spülung des erfindungsgemäßen Atemgerätes 1 erfolgt unter Verwendung des Umschaltventils 10 und des Umschaltventils für die Frischgaszufuhr 18, wobei das Ausatemgas in den Fortgasströmungspfad 15 geleitet wird. Das Umschaltventil 16 dient in diesem Zusammenhang neben der Wiedergewinnung und Rückführung der überschüssigen Anästhesiegase auch zur Druckentlastung beim Übersteigen des PEEP-Sollwerts. Die Umschaltung zwischen den verschiedenen Betriebsmodi, respektive Handbetrieb oder Respiratorbetrieb, des Atemgerätes 1 erfolgt mittels des Umschaltventils Hand-/Respiratorbetrieb 3, welches mit einem Druckbegrenzungsventil 24 für die Handbeatmung bzw. den Handbetrieb zusammenwirkt. Das Umschaltventil Hand-/Respiratorbetrieb 3 und das nachgeschaltete Druckbegrenzungsventil sind im sogenannten aber nicht näher bezeichneten Handbeatmungszweig platziert, welche direkt in den Fortgasströmungspfad 15 mündet.

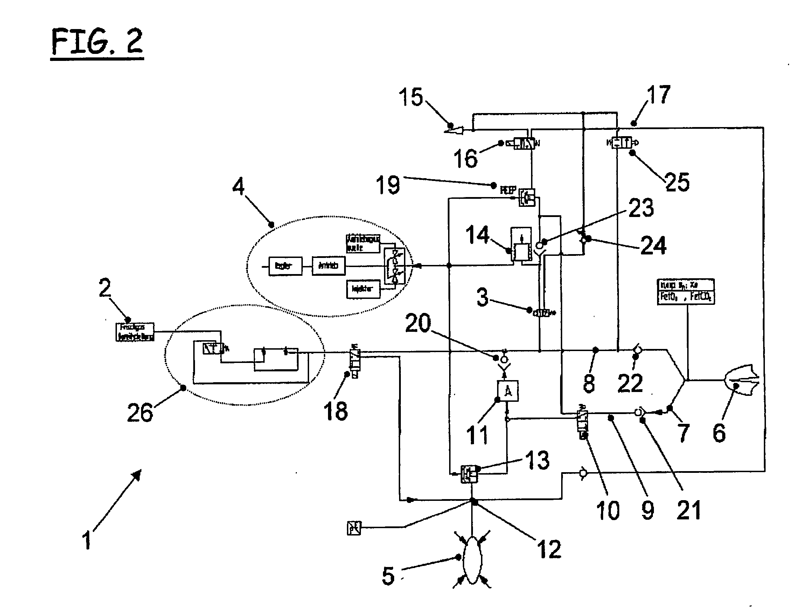

Die Fig. 2 zeigt das zuvor beschriebene erfindungsgemäße Atemgerät 1 als halbgeschlossenes Atemgerät 1 in der Exspirationsphase als Respirator Beatmung. Die als Rückschlagventile ausgebildeten Ventile 20, 21 und 23 sind dabei geöffnet und das als Rückschlagventil ausgebildete Ventil 22 im Inspirationszweig 8 ist geschlossen.

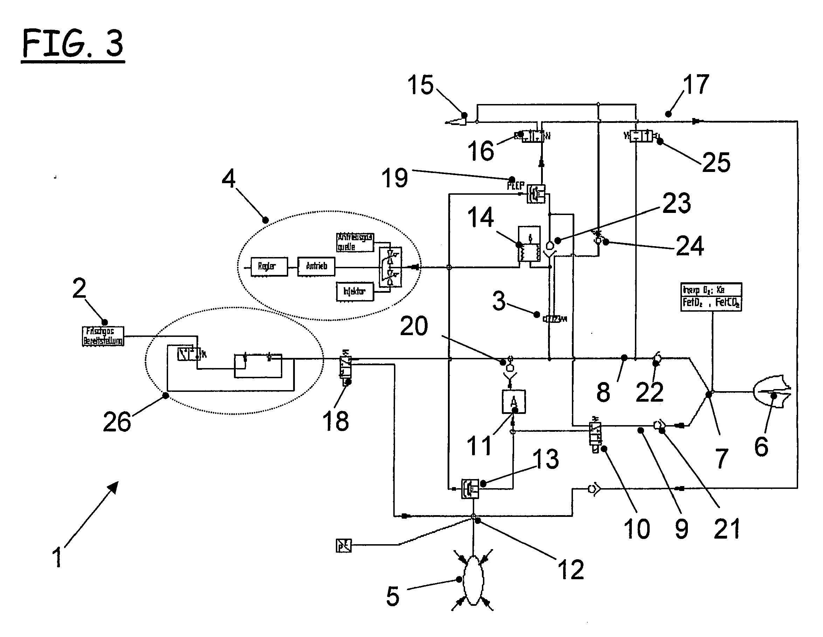

Der Fig. 3 ist das in der Fig. 1 beschriebene erfindungsgemäße Atemgerät 1 als geschlossenes Atemgerät in der Exspirationsphase als Respirator-Beatmung zu entnehmen. Der Balg des Domes 14 hebt sich in der Exspirationsphase, da Luft aus dem den Balg umgebenden Dom herausgesaugt wird. Die Rückschlagventile 20, 21 und 23 sind dabei geöffnet und das Rückschlagventil 22 im Inspirationszweig 8 ist geschlossen.

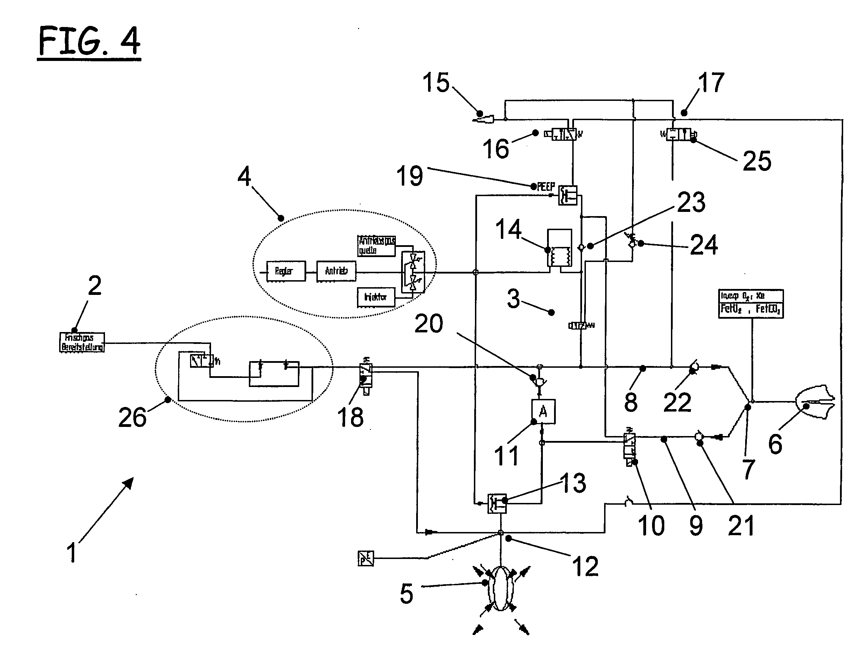

Fig. 4 illustriert das in der Fig. 1 beschriebene erfindungsgemäße Atemgerät 1 in der In - Exspirationsphase bei der Hand-Beatmung durch das als Handbeatmungsbeutel ausgebildete Gasreservoir 5. Das Rückschlagventil 22 ist dabei geschlossen und die Rückschlagventile 20 und 21 sind geöffnet. Die Umschaltung in den Handbetrieb erfolgt mittels des Umschaltventils 3, so dass der Respiratorbetrieb unterbunden ist. Die exspiratorische Patientenluft hat keine Verbindung zu dem innerhalb des Doms 14 angeordnete Balg. Der gewünschte Druck und das überschüssige Anästhesiegas wird durch das manuelle Druckbegrenzungsventil 24 eingestellt. Das überschüssige Anästhesiegas wird dann zum Fortgasströmungspfad 15 weitergeleitet. Bei der Handbeatmung bzw. im Handbetrieb sollte aus Sicherheitsgründen keine Rückkopplung von exspiratorischem Atemgas mit dem als Handbeatmungsbeutel ausgebildete Gasreservoir 5 erfolgen. Ein etwaiger Überschuss an Anästhesiegase kann dadurch sehr gering gehalten werden, in dem man das als Handbeatmungsbeutel ausgebildete Gasreservoir 5 moderat betätigt und der Druck am Druckbegrenzungsventil 24 höher eingestellt wird.

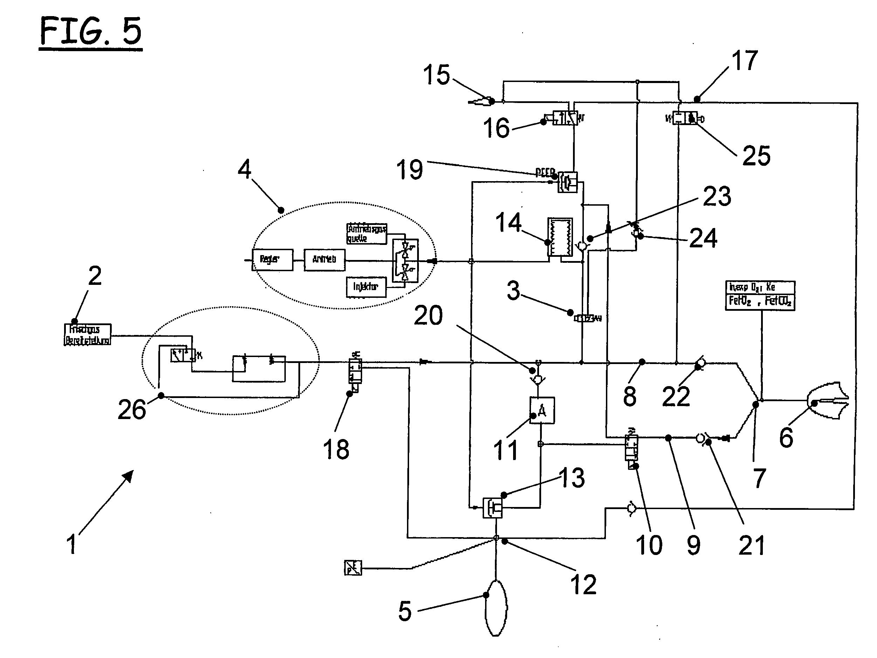

Die Fig. 5 zeigt das in der Fig. 1 beschriebene erfindungsgemäße Atemgerät 1 in der Ausbildung als halboffenes Atemgerät in der Exspirationsphase als Respirator-Beatmung. Die Rückschlagventile 20 und 22 sind dabei geschlossen und das Rückschlagventil 21 im Exspirationszweig 9 ist geöffnet. Bei diesem halboffenen Atemgerät 1 wird das Umschaltventil 10 derart angesteuert und geschaltet, dass der innerhalb des Doms 14 angeordnete Balg unter Verwendung des Rückschlagventils 23 im Exspirationszweig 9 umgangen wird. Bei einem halboffenen Atemgerät 1 darf der Patient nicht in den Balg des Doms 14 ausatmen, so dass das exspiratorische Atemgas vollständig dem Rückführungsströmungspfad 15 zugeleitet wird. Der CO₂-Absorber 11 wird hierbei nicht in Anspruch genommen. Die bei einem halboffenen System große Menge an benötigtem Frischgas wird bei der Narkose zu schnellen Gasanreicherung (Spülung), bei einem Atemkalkwechsel oder wenn kein Atemkalk vorhanden ist, verwendet.

### LISTE DER BEZUGSZEICHEN

- 1: Atemgerät
- 2: Frischgasbereitstellung
- 3: Umschaltventil Hand-/Respiratorbetrieb
- 4: Antriebsgasbereitstellung
- 5: Gasreservoir
- 6: Lunge des Patienten
- 7: Y-Stück
- 8: Inspirationszweig
- 9: Exspirationszweig
- 10: Umschaltventil
- 11: CO₂-Absorber
- 12: Mischpunkt
- 13: Frischgasentkopptungsventil
- 14: Dom
- 15: Fortgasströmungspfad
- 16: Umschaltventil
- 17: Rückführungsströmungspfad
- 18: Umschaltventil für Frischgaszufuhr
- 19: Membranventil für PEEP-Regelung
- 20: Rückschlagventil
- 21: Rückschlagventil
- 22: Rückschlagventil
- 23: Rückschlagventil
- 24: Druckbegrenzungsventil für Handbetrieb
- 25: Notentlüftungsventil
- 26: Druckhalteeinrichtung

## Patentansprüche

1. Atemgerät (1), insbesondere ein kombiniertes Narkose- und Therapiebeatmungsgerät, zumindest aufweisend eine Frischgasbereitstellung (2), eine Antriebsgasbereitstellung (4), ein Y-Stück (7) mit einem Inspirationszweig (8) und einem Exspirationszweig (9) sowie zugehörige Steuer- und Regeleinrichtungen, wobei zum Zwecke der Rückgewinnung und Rückführung von überschüssigen Anästhesiegas ein im Exspirationszweig (9) platziertes steuerbares Umschaltventil (16) mit einem Rückführungsströmungspfad (17) und einem Fortgasströmungspfad (15) sowie ein im Rückführungsströmungspfad (17) platziertes und in den Inspirationszweig (8) mündendes Gasreservoir (5) vorgesehen sind, **dadurch gekennzeichnet, dass** dem Gasreservoir (5) in Gasströmungsrichtung ein Frischgasentkopplungsventil (13) nachgeschaltet ist, welches sowohl mit dem Gasreservoir als auch mit der Frischgasbereitstellung (2) gekoppelt ist.

2. Atemgerät (1) nach Anspruch 1, **dadurch gekennzeichnet, dass** zwischen dem Gasreservoir (5) und dem Frischgasentkopplungsventil (13) ein Mischpunkt (12) platziert ist, welcher sowohl mit dem Gasreservoir als auch mit der Frischgasbereitstellung (2) verbunden ist.

3. Atemgerät (1) nach Anspruch 1 oder 2, **dadurch gekennzeichnet, dass** das Umschaltventil (16) zur Freigabe des Fortgasströmungspfades (15) oder zur Freigabe des Rückführungsströmungspfads (17) zum Zwecke der Umschaltung des Atemgerätes (1) in ein halboffenes oder ein geschlossenes System zwischen seinen Endlagen positionierbar und steuerbar ausgebildet ist.

4. Atemgerät, (1) nach einem der Ansprüche 1 bis 3, **dadurch gekennzeichnet, dass** das Gasreservoir (5) zum Zwecke der Beatmung durch Handkraft als Handbeatmungsbeutel ausgebildet ist.

5. Atemgerät, (1) nach einem der Ansprüche 1 bis 4, **dadurch gekennzeichnet, dass** zur Absorbierung des CO₂ des in das Atemgerät einströmenden Frischgases und/oder des rückströmenden und wiedergewonnenen Anästhesiegases ein CO₂-Absorber (11) vorgesehen ist.

6. Verfahren zum Betrieb des Atemgerätes (1) nach einem der Ansprüche 1 bis 5, **dadurch gekennzeichnet, dass** der als Indikator für den Füllungsgrad im Atemgerät (1) verwendete exspiratorische Druck (PEEP) des Atemgerätes- (1) geregelt wird, wobei als Störgrößen der Frischgaszustrom - definiert als Stellung eines Gasmischers - sowie der Fortgasstrom - definiert als Stellung eines Umschaltventils (16) und eines PEEP-Ventils (19) - eingesetzt werden.

7. Verfahren zum Betrieb des Atemgerätes (1) nach Anspruch 6, **dadurch gekennzeichnet, dass** der erfassbare Erschöpfungsgrad des Gasreservoirs (5) zur Steuerung des an den Rückstrom des wiedergewonnenen Anästhesiegases angepassten Frisdigaszustroms eingesetzt wird.

8. Verfahren zum Betrieb des Atemgerätes (1) nach Anspruch 6 oder 7, **dadurch gekennzeichnet, dass** in das Atemgerät (1) einströmende Frischgas und das rückströmende und wiedergewonnene Anästhesiegas am Mischpunkt (12) ihrer Strömungswege oder im Frischgasentkopplungsventil (13) einer Durchmischung unterzogen werden.

9. Verfahren zum Betrieb des Atemgerätes (1) nach einem der Ansprüche 6 bis 8, **dadurch gekennzeichnet, dass** eine Spülung des Atemgerätes (1) durch
a. die Freigabe des Fortgasströmungspfads (15) des Umschaltventils (16), die Steuerung der Umschaltventile (10), (18) und damit die Umschaltung in ein halbgeschlossenes Atemgerät, (1) und durch
b. die Einstellung einer veränderten Konzentrationsvorgabe von O₂ und Xe am Gasmischer
realisiert wird.

## Claims

1. Ventilator apparatus (1), in particular a combined anaesthetic and therapeutic ventilator apparatus with at least one fresh gas supply (2), one deriving gas supply unit (4), a Y-shaped piece (7) with an inspiration branch (8) and an expiration branch (9) as well as associated control and regulation devices, with a controllable switching valve (16), fitted in the expiration branch (9) and provided with a return flow path (17) and an exhaust gas flow path (15), as well as a gas reservoir (5) positioned in the return flow path (17) and opening into the inspiration branch (8) in order to recover and recirculate excess anaesthetic gas, **characterised by that** a fresh gas decoupling valve (13) is connected downstream of the gas reservoir (5) in the gas flow direction, which valve is connected both to the gas reservoir and to the fresh gas supply (2).

2. Ventilator apparatus (1) according to claim 1, **characterised by that** a mixing point (12) is positioned between the gas reservoir (5) and the fresh gas decoupling valve (13), which is connected both to the gas reservoir and to the fresh gas supply (2).

3. Ventilator apparatus (1) according to claim 1 or 2, **characterised by that** the switching valve (16) for releasing the exhaust gas flow path (15) or for releasing the return flow path (17) is designed to be positioned and controlled between its end positions in order for the ventilator apparatus (1) to be switched to a half-open or closed system.

4. Ventilator apparatus (1) according to one of the claims 1 to 3, **characterised by that** the gas reservoir (5) is designed as a hand ventilation bag for ventilation by hand force.

5. Ventilator apparatus (1) according to one of the claims 1 to 4, **characterised by that** a CO₂ absorber (11) is provided in order to absorb the CO₂ in the fresh gas flowing into the ventilator apparatus and/or in the returning and recovered anaesthetic gas.

6. Method for operating the ventilator apparatus (1) according to one of the claims 1 to 5, **characterised by** that the expiratory pressure (PEEP) of the ventilator apparatus (1), used as an indicator for the full level of the ventilator apparatus (1), is controlled, the control variables being the fresh gas inflow - defined as the position of a gas mixer - and the exhaust gas flow - defined as the position of a switching valve (16) and a PEEP valve (19).

7. Method for operating the ventilator apparatus (1) according to claim 6, **characterised by** that the recordable level of consumption of the gas reservoir (5) is used to control the fresh gas flow adjusted to the recirculation of the recovered anaesthetic gas.

8. Method for operating the ventilator apparatus (1) according to claim 6 or 7, **characterised by** that fresh gas and recirculated and recovered anaesthetic gas flowing into the ventilator apparatus (1) are mixed at the mixing point (12) of their flow paths or in the fresh gas decoupling valve (13).

9. Method for operating the ventilator apparatus (1) according to one of the claims 6 to 8, **characterised by** that the ventilator apparatus (1) is purged by:
a. Releasing the switching valve (16) in the exhaust gas flow path (15), controlling the switching valves (10), (18) and therefore by switching to a half-closed ventilator apparatus (1) and by
b. Setting an altered 02 and Xe concentration level on the gas mixer.

## Revendications

1. Appareil respiratoire (1), en particulier un respirateur d'anesthésie et de thérapie combiné, présentant au moins une alimentation en gaz frais (2), une alimentation en gaz d'entraînement (4), un raccord Y (7) avec une branche inspiratoire (8) et une branche expiratoire (9) ainsi que des dispositifs correspondants de commande et de réglage, en quoi une vanne d'inversion (16) gouvernable placée dans la branche expiratoire (9) avec une voie d'écoulement de retour (17) et une voie d'écoulement du gaz d'éjection (15) ainsi qu'un réservoir de gaz (5) placé dans la voie d'écoulement de retour (17) et débouchant dans la branche inspiratoire (8) sont prévus aux fins de récupération et de retour de gaz d'anesthésie excédentaire, **caractérisé en ce qu'**une vanne de découplage du gaz frais (13) est montée en aval du réservoir de gaz (5) dans le sens d'écoulement du gaz, laquelle vanne est couplée aussi bien avec le réservoir de gaz qu'avec l'alimentation en gaz frais (2).

2. Appareil respiratoire (1) selon la revendication 1, **caractérisé en ce qu'**un point de mélange (12) est placé entre le réservoir de gaz (5) et la vanne de découplage du gaz frais (13), lequel point de mélange est relié aussi bien avec le réservoir de gaz qu'avec l'alimentation en gaz frais (2).

3. Appareil respiratoire (1) selon la revendication 1 ou 2, **caractérisé en ce que** la vanne d'inversion (16) d'ouverture de la voie d'écoulement du gaz d'éjection (15) ou d'ouverture de la voie d'écoulement de retour (17) est formée pour être ancrable entre ses positions finales et gouvernable aux fins de passage de l'appareil respiratoire (1) à un système semi-ouvert ou fermé.

4. Appareil respiratoire (1) selon une des revendications 1 à 3, **caractérisé en ce que** le réservoir de gaz (5) est formé comme un ballon de ventilation à main aux fins de ventilation par la force manuelle.

5. Appareil respiratoire (1) selon une des revendications 1 à 4, **caractérisé en ce qu'**un absorbeur de CO₂ (11) est prévu pour l'absorption du CO₂ du gaz frais affluant dans l'appareil respiratoire et/ou du gaz d'anesthésie refluant et récupéré.

6. Processus d'exploitation de l'appareil respiratoire (1) selon une des revendications 1 à 5, **caractérisé en ce que** la pression expiratoire (PEEP) de l'appareil respiratoire (1) employée dans l'appareil respiratoire (1) comme indicateur du taux de remplissage est réglée, en quoi l'afflux de gaz frais - défini comme position d'un mélangeur de gaz - ainsi que écoulement du gaz d'éjection - défini comme position d'une vanne d'inversion (16) et d'une vanne PEEP (19) - sont utilisés comme variables correctrices.

7. Processus d'exploitation de l'appareil respiratoire (1) selon la revendication 6, **caractérisé en ce que** le stade d'épuisement enregistrable du réservoir de gaz (5) est utilisé pour la commande de afflux de gaz frais ajusté au reflux du gaz d'anesthésie récupéré.

8. Processus d'exploitation de l'appareil respiratoire (1) selon la revendication 6 ou 7, **caractérisé en ce que** le gaz frais affluant dans l'appareil respiratoire (1) et le gaz d'anesthésie refluant et récupéré sont soumis à un brassage au point de mélange (12) de leurs voies d'écoulement ou dans la vanne de découplage du gaz frais (13).

9. Processus d'exploitation de l'appareil respiratoire (1) selon une des revendications 6 à 8, **caractérisé en ce qu'**un rinçage de l'appareil respiratoire (1) est réalisé par
a. l'ouverture de la voie d'écoulement du gaz d'éjection (15) de la vanne d'inversion (16), la commande des vannes d'inversion (10), (18) et ainsi le passage à un appareil respiratoire (1) semi-fermé, et par
b. le réglage d'une directive de concentration modifiée d'O₂ et de Xe sur le mélangeur de gaz.
